# NEUE EUROPÄISCHE PATENTSCHRIFT

(11) **EP 1 255 720 B2**
(45) Veröffentlichungstag und Bekanntmachung des Hinweises auf die Entscheidung über den Einspruch: **15.04.2009**
(45) Hinweis auf die Patenterteilung: 10.09.2003
(21) Anmeldenummer: 01911637.5
(22) Anmeldetag: 13.02.2001
(51) Int. Cl.: C07C 45/50

(54) **VERFAHREN ZUR AUFARBEITUNG EINES FLÜSSIGEN HYDROFORMYLIERUNGSAUSTRAGES**
METHOD FOR PROCESSING A LIQUID HYDROFORMYLATION DISCHARGE
PROCEDE DE TRAITEMENT D'UNE DECHARGE LIQUIDE D'HYDROFORMYLATION

(30) Priorität: 14.02.2000 DE 10006489
(43) Veröffentlichungstag der Anmeldung: 13.11.2002
(73) Patentinhaber: BASF SE, 67056 Ludwigshafen (DE)
(72) Erfinder: WALCZUCH, Karl-Heinz, 67117 Limburgerhof (DE); MÜLLER, Rolf, 67125 Dannstadt-Schauernheim (DE); KROKOSZINSKI, Roland, 67273 Weisenheim a. Berg (DE); GEISSLER, Bernhard, 67281 Kirchheim (DE)
(74) Vertreter: Kinzebach, Werner
(86) Internationale Anmeldenummer: PCT/EP2001/001582
(87) Internationale Veröffentlichungsnummer: WO 2001/058844

(56) Entgegenhaltungen:
- WO-A-97/07086
- DE-A- 19 530 698
- US-A- 4 148 830
- US-A- 4 287 370
- US-A- 4 792 636
- US-A- 5 001 274
- US-A- 5 410 091

## Beschreibung

Die Erfindung betrifft ein Verfahren zur Aufarbeitung eines flüssigen Hydroformylierungsaustrages einer kontinuierlichen Hydroformylierungsreaktion, enthaltend mindestens einen Aldehyd als Hydroformylierungsprodukt, nicht umgesetzte Olefine, gelöstes Synthesegas, den homogen gelösten Hydroformylierungskatalysator sowie Nebenprodukte der Hydroformylierungsreaktion.

Die Hydroformylierung von Olefinen zu den entsprechenden Aldehyden ist von enormer wirtschaftlicher Bedeutung, da die auf diesem Wege hergestellten Aldehyde wiederum Ausgangsmaterial für eine Vielzahl großtechnischer Produkte, wie Lösemittel oder Weichmacheralkohole sind. Dementsprechend werden Hydroformylierungsverfahren weltweit stark beforscht, um beispielsweise die Energiebilanz des Verfahrens zu verbessern, die Selektivität zu erhöhen und den homogenen Rhodium-Katalysators schonender zu behandeln.

Für die Hydroformylierung von C₂ bis C₂₀-Olefinen wird im Allgemeinen das sogenannte Flüssigaustragsverfahren angewandt, wie es aus der EP-A-114 611, US-4148830 oder der EP-A-016 286 bekannt ist, wobei der im Wesentlichen flüssige Austrag aus der Hydroformylierungsreaktion in ein Entspannungsgefäß entspannt wird. Infolge der Druckerniedrigung wird der Austrag in eine flüssige, den Katalysator, Lösemittel, hochsiedende Nebenprodukte und eine geringe Menge an Aldehyd und nicht umgesetztem Olefin enthaltende flüssige Phase und eine neben dem überschüssigem Synthesegas den Hauptteil des gebildeten Aldehyds und des nicht umgesetzten Olefins enthaltende Gasphase aufgetrennt. Die flüssige Phase wird als Rückführstrom wieder in den Reaktor geleitet, und die Gasphase abgezogen. Die Gasphase wird aufgetrennt in das Synthesegas sowie die nicht umgesetzten Olefine und den Aldehyd, welcher destillativ vom nicht umgesetzten Olefin getrennt wird. Die Synthesegase sowie die nicht umgesetzten Olefine werden in den Reaktor zurückgeführt.

Die WO 97/07086 beschreibt ein modifiziertes Verfahren, bei dem die Flüssigphase des Entspannungsgefäßes in den oberen Teil einer Kolonne aufgegeben und die Gasphase in den unteren Teil der Kolonne geleitet wird, so dass die Flüssigphase mit der Gasphase im Gegenstrom behandelt wird. Dadurch wird die Trennung von Produkt und hochsiedenden Komponenten verbessert. Diese Trennung wird zweckmäßigerweise bei möglichst niedrigem Druck durchgeführt, um die Trennung von Produkt und Hochsiedern bei Temperaturen durchführen zu können, die den Katalysator nicht schädigen.

Der Nachteil dieses Verfahrens besteht darin, dass große Verdichter mit hohem Energieverbrauch eingesetzt werden müssen, um das überschüssige Synthesegas, nicht umgesetzte Olefine sowie leichtsiedende Nebenprodukte auf Reaktionsdruck zu komprimieren und wieder in den Reaktor zurückzuführen.

Der vorliegenden Erfindung lag deshalb die Aufgabe zugrunde, ein wirtschaftlicheres Verfahren zur Hydroformylierung von Olefinen zur Verfügung zu stellen, bei dem die oben genannten Nachteile bei der weiteren Aufarbeitung des flüssigen Hydroformylierungsaustrages aus dem Hydroformylierungsreaktor umgangen werden.

Überraschenderweise wurde nun gefunden, dass diese Aufgabe durch ein Verfahren gelöst wird, das einen zweistufigen Flash des Hydroformylierungsaustrages umfasst.

Gegenstand der Erfindung ist dementsprechend ein Verfahren zur Aufarbeitung eines flüssiges Austrages einer kontinuierlichen Hydroformylierung, der Aldehyde, hochsiedende Nebenprodukte, einen homogen gelösten Hydroformylierungskatalysator, nicht umgesetzte Olefine, leichtsiedende Nebenprodukte und gelöstes Synthesegas enthält, wobei
a) der flüssige Hydroformylierungsaustrag in einer ersten Entspannungsstufe auf einen Druck entspannt wird, der 2 bis 20 bar unterhalb des Reaktordruckes liegt und der Druck in dem Entspannungsgefäß im Bereich von 2 bis 40 bar liegt, wobei eine Auftrennung in eine Flüssigphase und eine Gasphase erfolgt und die in der ersten Entspannungsstufe anfallende Gasphase in den Reaktor zurückgeführt wird; und
b) die in der ersten Entspannungsstufe erhaltene Flüssigphase in einer zweiten Entspannungsstufe auf einen Druck entspannt wird, der niedriger als der Druck der ersten Entspannungsstufe ist, wobei eine Auftrennung erfolgt in eine Flüssigphase, die im Wesentlichen hochsiedende Nebenprodukte der Hydroformylierung, den homogen gelösten Hydroformylierungskatalysator und geringe Mengen an Hydroformylierungsprodukt und unumgesetztem Olefin enthält und in eine Gasphase, die im Wesentlichen die Hauptmenge des Hydroformylierungsproduktes, nicht umgesetztes Olefin und leichtsiedende Nebenprodukte enthält.

Das erfindungsgemäße Verfahren eignet sich für die Aufarbeitung von Flüssigausträger aus der Rhodium-katalysierten Hydroformylierung von Olefinen. Als Olefine kommen im Allgemeinen solche mit 2 bis 20 Kohlenstoffatomen, insbesondere 2 bis 10 Kohlenstoffatomen und besonders bevorzugt 2 bis 5 Kohlenstoffatomen, oder Gemische davon zur Anwendung. Die eingesetzten Olefine können unsubstituiert sein oder einen oder zwei unter den Hydroformylierungsbedingungen inerten Substituenten aufweisen, beispielsweise eine Estergruppe, Nitrilgruppe, Alkoxygruppe oder Hydroxygruppe.

Bei den als Katalysatoren verwendeten Rhodium-Katalysatoren handelt es sich im Allgemeinen um homogen im Reaktionsmedium der Hydroformylierungsreaktion lösliche Komplexe mit einer oder mehreren Organophosphorverbindungen als Liganden. Beispiele für solche Liganden sind Phosphinliganden aus der Klasse der Triarylphosphine, insbesondere Triphenylphosphin, C₁ - C₆ - Alkyldiarylphosphine oder Arylalkyldiphosphine. Brauchbare Katalysatoren sind beispielsweise in der WO 97/07086 sowie in den darin genannten Patentpublikationen beschrieben.

Die Hydroformylierung wird im Allgemeinen bei einer Temperatur im Bereich von 50 bis 150 °C und einem Druck im Bereich von 5 bis 50 bar durchgeführt.

Bei der angegebenen Temperatur und dem angegebenen Druck ist das im Überschuss zur Hydroformylierung eingesetzte Synthesegas - ein Kohlenmonoxid/Wasserstoff-Gemisch mit einem CO/H₂-Molverhältnis von im Allgemeinen 20/80 bis 80/20, vorzugsweise von 40/60 bis 60/40 entsprechend seiner Löslichkeit im flüssigen Hydroformylierungsaustrag gelöst. Ein Teil des Synthesegases kann in Form von kleinen Gasblasen im Hydroformylierungsaustrag suspendiert sein.

Der flüssige Teil des Austrags aus der Hydroformylierungsreaktion enthält als wesentlichen Bestandteil den Rhodium-Katalysator, das Hydroformylierungsprodukt, also den oder die aus dem eingesetzten Olefin oder Olefingemischen erzeugten Aldehyde und weiterhin höhere als das Hydroformylierungsprodukt siedende Kondensationsprodukte dieser Aldehyde, wie sie als Nebenprodukte im Zuge der Hydroformylierung entstehen können und beispielhaft in der US 4,158,830 beschrieben worden sind, sowie leichtsiedende Komponenten, wie insbesondere die den Olefinen entsprechenden Alkane. Gegebenenfalls enthält der Flüssigaustrag auch ein hochsiedendes, inertes Lösungsmittel, wie Toluol oder Xylol.

Die voranstehenden Darlegungen zum Hydroformylierungsverfahren und dem verwendeten Rhodium-Katalysator dienen dazu, das erfindungsgemäße Verfahren erläuternd in seinem technischen Gesamtzusammenhang zu stellen. Es sei an dieser Stelle erwähnt, dass die dem erfindungsgemäßen Verfahren vorausgehende Hydroformylierung nach an sich bekannten und gebräuchlichen Hydroformylierungsverfahren mit Flüssigaustrag des Standes der Technik, beispielsweise nach der EP-A-0 16 286, EP-A-188 246 oder der US 4,148,830, durchgeführt werden kann.

Vorzugsweise wird der flüssige Hydroformylierungsaustrag zunächst auf eine Temperatur erwärmt, die um 5 bis 50 °C, vorzugsweise 10 bis 30 °C über der Reaktortemperatur liegt. Das Erwärmen erfolgt auf übliche Weise, im Allgemeinen mittels Wärmetauscher.

Der gegebenenfalls erwärmte Hydroformylierungsaustrag wird dann in einer ersten Entspannungsstufe in einen Behälter (Entspannungsgefäß) auf einen Druck entspannt, der 2 bis 20 bar, vorzugsweise 5 bis 15 bar, unterhalb des Reaktordruckes liegt. Der Druck in dem Entspannungsgefäß liegt dann im Bereich von 2 bis 40 bar, vorzugsweise 2 bis 20 bar.

In der ersten Entspannungsstufe wird der Hydroformylierungsaustrag in eine Flüssigphase und eine Gasphase aufgetrennt. Die Gasphase enthält im Wesentlichen überschüssiges Synthesegas, unumgesetztes Olefin und gegebenenfalls das dem Olefin entsprechende Alkan. Die Gasphase wird, üblicherweise nach Komprimierung auf den Reaktordruck, wieder in den Reaktor zurückgeführt. Die Flüssigphase enthält im Wesentlichen das Hydroformylierungsprodukt, höher siedende Kondensationsprodukte des Hydroformylierungsproduktes, den Katalysator und gegebenenfalls ein Lösungsmittel, wie Toluol oder Xylol.

Die in der ersten Entspannungsstufe abgeschiedene flüssige Phase wird dann als flüssiger Strom aus dem Entspannungsgefäß ausgetragen und in einer zweiten Entspannungsstufe in ein weiteres Entspannungsgefäß auf einen Druck entspannt, der niedriger als der Druck der ersten Entspannungsstufe ist. Vorzugsweise wird in der zweiten Entspannungsstufe auf einen Druck entspannt, der im Bereich von 0 bis 10 bar, vorzugsweise 1 bis 5 bar, liegt. Der Druck in der zweiten Entspannungsstufe liegt im Allgemeinen um 2 bis 20 bar, insbesondere 3 bis 15 bar, niedriger als der Druck in der ersten Entspannungsstufe.

Die aus der ersten Entspannungsstufe erhaltene Flüssigphase wird in der zweiten Entspannungsstufe in eine Flüssigphase und eine Gasphase aufgetrennt. Die Flüssigphase enthält die hochsiedenden Kondensationsprodukte des Hydroformylierungsproduktes, den Katalysator sowie gegebenenfalls Lösungsmittel und geringe Mengen an Hydroformylierungsprodukten. Die Gasphase enthält die Hauptmenge an Hydroformylierungsprodukt sowie Reste an Synthesegas und leichtsiedenden Komponenten (unumgesetzes Olefin und dem Olefin entsprechendes Alkan).

Überraschenderweise wurde gefunden, dass sowohl der Energieverbrauch als auch die Anforderungen an die Kapazität des Verdichters für die Komprimierung von überschüssigem Synthesegas und unumgesetzem Olefin reduziert werden, wenn erfindungsgemäß eine zweistufige Entspannung des Hydroformylierungsaustrages vorgenommen wird.

Die in der zweiten Entspannungsstufe anfallende Flüssigphase und Gasphase können nach üblichen Verfahren weiter aufgearbeitet werden. Beispielsweise kann die Gasphase einem Kondensator zugeführt werden, in welchem das Hydroformylierungsprodukt und noch vorhandenes, nicht umgesetztes Olefin sowie leichtsiedende Komponenten (in erster Linie das dem Olefin entsprechende Alkan) flüssig abgeschieden und der weiteren Aufreinigung, z. B. durch Destillation, zugeführt werden. Die in dem Kondensator anfallende Gasphase, die im Wesentlichen nicht umgesetztes Synthesegas sowie nicht umgesetztes Olefin und leichtsiedende Nebenkomponenten enthält, kann ganz oder teilweise in den Reaktor zurückgeführt werden.

Die in der zweiten Entspannungsstufe anfallende Flüssigphase kann direkt oder nach Entfernung des noch enthaltenen Formylierungsproduktes, z. B. durch Destillation, wieder in den Reaktor zurückgeführt werden.

Vorzugsweise wird die in der zweiten Entspannungsstufe anfallende Gas- und Flüssigphase nach dem in der WO 97/07086 beschriebenen Verfahren aufgearbeitet. Zu diesem Zweck wird die Flüssigphase in den oberen Bereich einer Kolonne eingeleitet, während die Gasphase in den Sumpf der Kolonne eingeleitet wird. Flüssigphase und Gasphase werden dadurch im Gegenstrom behandelt. Um den Kontakt zwischen Flüssigphase und Gasphase zu erhöhen, wird vorzugsweise eine Kolonne verwendet, die mit Füllkörpern, wie Raschig-Ringen, Spiralen oder Sattelkörpern, oder Packungen oder Einbauten, wie Rieselböden, ausgestattet ist, um eine große Oberfläche zu schaffen. Durch den innigen Kontakt der Gasphase mit der Flüssigphase werden die in der Flüssigphase vorhandenen Restmengen an Hydroformylierungsprodukt und nicht umgesetztem Olefin in die Gasphase transferiert, so dass der die Kolonne am Kopf verlassende Gasstrom im Vergleich zu dem am unteren Ende der Kolonne eingeführten Gasstrom an Hydroformylierungsprodukt und nicht umgesetztem Olefin angereichert ist. Die weitere Aufarbeitung des die Kolonne verlassenden Gasstroms und der die Kolonne verlassenden Flüssigphase erfolgt dann in üblicher Weise, beispielsweise wie bereits oben beschrieben.

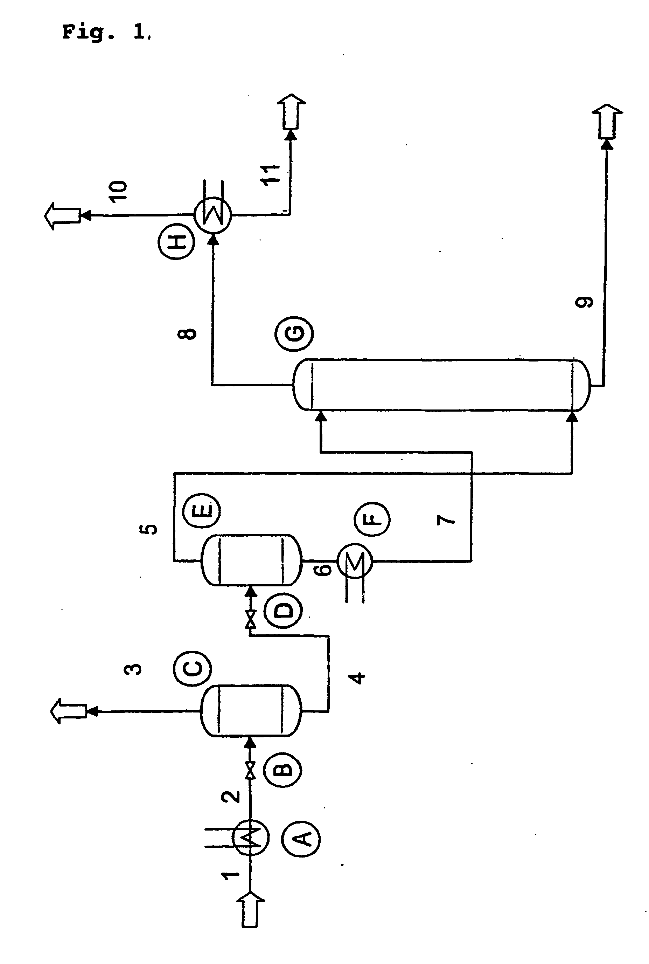

Das bevorzugte erfindungsgemäße Verfahren wird im Folgenden unter Zuhilfenahme der beigefügten Zeichnung unter Verwendung der darin angegebenen Bezugszeichen erläutert. Die Zeichnung ist allein ein der Erläuterung des erfindungsgemäßen Verfahren dienendes, schematisches Verfahrensbild, in dem, aus Gründen der Übersichtlichkeit, nur die für die Erläuterung des Verfahrens notwendigen Vorrichtungen eingezeichnet sind, wohingegen andere für die Durchführung des Verfahrens notwendige und selbstverständliche Vorrichtungen, wie Pumpen, zusätzliche Ventile, Mess- und Regeleinrichtungen usw. in der Zeichnung weggelassen wurden. Das erfindungsgemäße Verfahren ist nicht auf die in der Zeichnung dargestellte Ausführungsform beschränkt.

Der Hydroformylierungsaustrag (1) wird im Wärmetauscher (A) auf eine Temperatur erwärmt, die um maximal 50 °C über der Reaktortemperatur liegt. Die Erwärmung des Reaktoraustrags ist bevorzugt, das Verfahren kann jedoch auch ohne diese Erwärmung durchgeführt werden. Der erwärmte Hydroformylierungsaustrag (2) wird über ein Ventil (B) in den Behälter (C) (erstes Entspannungsgefäß) entspannt. Im Behälter (C) herrscht ein Druck, der 2 bis 20 bar unterhalb des Druckes des Hydroformylierungsaustrages (1) liegt. In dem Behälter (C) erfolgt eine Auftrennung in eine Gasphase, welche die Hauptmenge an überschüssigem Synthesegas, nicht umgesetzten Olefinen und leichtsiedenden Nebenprodukten enthält und eine Flüssigphase. Die im Behälter (C) anfallende flüssige Phase (4) wird über das Regelventil (D) in den Behälter (E) (zweites Entspannungsgefäß) geleitet und entspannt. Durch die Entspannung in den Behälter (E) erfolgt eine Auftrennung in eine flüssige Phase und eine Gasphase. Die flüssige Phase enthält im Wesentlichen den Katalysator, höher siedende Nebenprodukte der Hydroformylierungsreaktion, Restmengen an Olefin und an Hydroformylierungsprodukt und gegebenenfalls ein bei der Hydroformylierung verwendetes, hochsiedendes Lösemittel. Die Gasphase enthält im Wesentlichen den Hauptteil des Hydroformylierungsproduktes und den Rest an nicht umgesetztem Olefin, leichtsiedenden Komponenten und nicht umgesetztem Synthesegas.

Die im Behälter (E) abgeschiedene flüssige Phase (6) wird abgezogen und über einen Durchlauferhitzer oder Wärmetauscher (F) auf eine Temperatur erhitzt, die 10 °C bis 80 °C über der Temperatur der flüssigen Phase im Behälter (E) liegt.

Die aufgeheizte, flüssige Phase (7) aus dem Behälter (E) wird über eine Leitung dem Kopfteil oder oberen Teil der Kolonne (G) zugeführt. Die im Behälter (E) erhaltene Gasphase (5) wird in den Sumpf der Kolonne (G) geleitet. Bei der Kolonne (G) handelt es sich um eine übliche Kolonne, die z. B. mit Füllkörpern, Packungen oder Einbauten für intensiven Gas/Flüssigkeitsaustausch bestückt ist. Der die Kolonne (G) am Sumpf verlassende flüssige Strom (9), der im Wesentlichen den Katalysator und höher als das Hydroformylierungsprodukt siedende Nebenprodukte der Hydroformylierungsreaktion, gegebenenfalls ein zusätzlich zur Hydroformylierung verwendetes, hochsiedendes Lösemittel sowie Restmengen an Aldehyden enthält, wird wieder in den Hydroformylierungsreaktor (nicht in der Zeichnung eingezeichnet) zurückgeführt. Der am Kopf der Kolonne (G) abgezogene Gasstrom (8), der das Hydroformylierungsprodukt sowie Restmengen an leichtsiedenden Komponenten und nicht umgesetztem Olefin und Synthesegas enthält, wird zur Abkühlung einem Kondensator (H) zugeführt, in dem eine Auftrennung in eine Flüssigphase (11) und eine Gasphase (10) vorgenommen wird. Die Flüssigphase (11) enthält das Hydroformylierungsprodukt und geringe Mengen an nicht umgesetztem Olefin und leichtsiedenden Komponenten und wird üblicherweise einer Destillation zur weiteren Aufreinigung zugeführt. Die Gasphase (10) enthält das restliche Synthesegas sowie nicht umgesetztes Olefin und leichtsiedende Nebenkomponenten. Die Gasphase wird nach Komprimierung auf den Druck der Hydroformylierunggsreaktion wieder in den Hydroformylierungsreaktor zurückgeführt. Zweckmäßigerweise wird ein Teil der Ströme (9) und (10) ausgeschleust, um eine Anreicherung an störenden Nebenkomponenten zu vermeiden.

Die nachfolgenden Beispiele erläutern die Erfindung, ohne sie zu. begrenzen.

### Beispiele

### Vergleichsbeispiel

Ein Reaktor zur Produktion von 10 kg/h Butyraldehyd aus Propen, Kohlenmonoxid, Wasserstoff und Rhodium-Triphenylphosphin-Katalysator wurde bei einer Temperatur von 90 °C und einem Druck von 20 bar betrieben. Aus dem Reaktor wurde eine Menge von 24 kg/h an flüssigem Reaktorinhalt abgezogen und in einem Austragssystem gemäß der WO 97/07086 weiterverarbeitet. Diesem Verfahren ist gegenüber dem erfindungsgemäßen Verfahren kein Wärmetauscher und kein weiterer Entspannungsbehälter vorgeschaltet, d. h. der Flüssigaustrag (1) wird direkt in den Behälter (E) entspannt. Entsprechend dem Verfahren der WO 97/07086 wurde der Flüssigaustrag in den Behälter auf einen Druck von 1,5 bar entspannt.

Durch die Entspannung des flüssigen Hydroformylierungsaustrages in den Entspannungsbehälter (E) wurde eine Auftrennung des im Wesentlichen flüssigen Hydroformylierungsaustrages in eine flüssige Phase und eine Gasphase bewirkt. Die flüssige Phase enthält im Wesentlichen den Katalysator und höhersiedende Nebenprodukte der Hydroformylierungsreaktion, Restmengen an Olefin und Hydroformylierungsprodukt. Die Gasphase enthält im Wesentlichen den Hauptteil des Hydroformylierungsproduktes, den Hauptteil des nicht umgesetzten Olefins, leichtsiedende Nebenkomponenten und nicht umgesetztes Synthesegas.

Die im Entspannungsbehälter (E) abgeschiedene flüssige Phase wurde als flüssiger Strom (6) aus dem Entspannüngsbehälter über eine Leitung abgezogen und über einen Durchlauferhitzer oder Wärmetauscher (F) auf eine Temperatur erhitzt, die 25 °C über der Temperatur der flüssigen Phase des Entspannungsbehälters (E) lag. Der so aufgeheizte, flüssige Strom (7) wurde über eine Leitung dem Kopfteil der Kolonne (G) zugeführt. Bei der Kolonne (G) handelte es sich um eine mit Pallringen bestückte Füllkörperkolonne mit einer theoretischen Trennstufenzahl von 5. Die Gasphase aus dem Entspannungsbehälter (E) wurde als Strom (5) in den Sumpf der Kolonne (G) und somit im Gegenstrom zu dem flüssigen Strom (7) geleitet. Der die Kolonne (G) am Sumpf über eine Leitung verlassende, an Hydroformylierungsprodukt und nicht umgesetztem Olefin abgereicherte Flüssigkeitsstrom (9), der im Wesentlichen den Katalysator und höher als das Hydroformylierungsprodukt siedende Nebenprodukte der Hydroformylierungsreaktion enthielt, wurde ganz oder teilweise wieder in den Hydroformylierungsreaktor (nicht in der Zeichnung eingezeichnet) zurückgeführt. Der am Kopf der Kolonne (G) über eine Leitung abgezogene, mit dem Hydroformylierungsprodukt und nicht umgesetztem Olefin angereicherte Gasstrom (8), der als zusätzliche nennenswerte Bestandteile gesättigte Kohlenwasserstoffe und nicht umgesetztes Synthesegas enthielt, wurde zur weiteren Aufarbeitung einem Kondensator (H) zugeführt, in dem die höhersiedenden Bestandteile - das Hydroformylierungsprodukt und geringe Mengen an nicht umgesetztem Olefin und leichtsiedenden Komponenten - durch Kondensation vom nicht umgesetzten Synthesegas abgetrennt wurde. Das so abgetrennte Synthesegas wurde nach Komprimierung auf den Druck der Hydroformylierungsreaktion wieder in den Hydroformylierungsreaktor zurückgeführt.

Mit dieser Austragungsvariante fielen nach der Kondensation im Wärmetauscher (H) noch 1,0 Normkubikmeter Gas an. Hierbei handelte es sich um nicht umgesetztes Synthesegas, Propan und Propen, welches im Reaktoraustrag gelöst war. Diese Gasmenge wurde über einen Verdichter auf Reaktionsdruck komprimiert und in den Reaktor zurückgeführt.

### Beispiel 1

Der Reaktoraustrag (1) wurde nach dem in der Figur beschriebenen Verfahren, jedoch ohne Verwendung des Wärmetauschers (A), aufgearbeitet. Ein großer Teil (ca. 40 Vol.-%) der gelösten Gase (Synthesegas, Propen und Propan) wurde in die Gasphase überführt. Diese Gase wurden direkt in den Reaktor zurückgeführt. Die verbleibende flüssige Phase wurde aus dem Behälter (C) über eine Leitung mit Regelventil (D) dem Entspannungsbehälter (E) zugeführt und gemäß dem Vergleichsbeispiel weiter behandelt.

Die nun nach dem Wärmetauscher (H) zurück zu verdichtende Gasmenge reduzierte sich von 1,0 auf 0,6 Normkubikmeter pro Stunde.

### Beispiel 2

Das Beispiel 1 wurde wiederholt, jedoch unter Verwendung des Wärmetauschers (A), im welchem der flüssige Reaktoraustrag von 90 °C auf 110 °C erwärmt wurde. Dadurch stieg nach der Entspannung auf circa 6 bar die Gasmenge (3) (ca. 60 Vol.-% der gelösten Gase), die aus dem Behälter (C) direkt in den Reaktor zurückgeführt wurde.

Die nun nach dem Wärmetauscher (H) zurück zu verdichtende Gasmenge reduzierte sich von 1,0 auf 0,4 Normkubikmeter pro Stunde.

## Patentansprüche

1. Verfahren zur Aufarbeitung eines flüssiges Austrages einer kontinuierlichen Hydroformylierung, der im Wesentlichen Aldehyde, hochsiedende Nebenprodukte, einen homogen gelösten Hydroformylierungskatalysator, nicht umgesetzte Olefine, leichtsiedende Nebenprodukte und gelöstes Synthesegas enthält, wobei
a) der flüssige Hydroformylierungsaustrag in einer ersten Entspannungsstufe auf einen Druck entspannt wird, der 2 bis 20 bar unterhalb des Reaktordruckes liegt und der Druck in dem Entspannungsgefäß im Bereich von 2 bis 40 bar liegt, wobei eine Auftrennung in eine Flüssigphase und eine Gasphase erfolgt und die in der ersten Entspannungsstufe anfallende Gasphase in den Reaktor zurückgeführt wird; und
b) die in der ersten Entspannungsstufe erhaltene Flüssigphase in einer zweiten Entspannungsstufe auf einen Druck entspannt wird, der niedriger als der Druck der ersten Entspannungsstufe ist, wobei eine Auftrennung erfolgt in eine Flüssigphase, die im Wesentlichen hochsiedende Nebenprodukte der Hydroformylierung, den homogen gelösten Hydroformylierungskatalysator und geringe Mengen an Hydroformylierungsprodukt und unumgesetztem Olefin enthält und in eine Gasphase, die im Wesentlichen die Hauptmenge des Hydroformylierungsproduktes, nicht umgesetztes Olefin und leichtsiedende Nebenprodukte enthält.

2. Verfahren nach Anspruch 1, wobei der Hydroformylierungsaustrag vor der ersten Entspannungsstufe auf eine Temperatur aufgeheizt wird, die um 5 bis 50 °C über der Reaktionstemperatur der Hydroformylierung liegt.

3. Verfahren nach Anspruch 1 oder 2, wobei in der ersten Entspannungsstufe auf einen Druck entspannt wird, der im Bereich von 3 bis 40 bar liegt.

4. Verfahren nach einem der vorhergehenden Ansprüche, wobei die in der ersten Entspannungsstufe erhaltene Flüssigphase in der zweiten Entspannungsstufe auf einen Druck im Bereich von 0 bis 10 bar entspannt wird.

5. Verfahren nach einem der vorhergehenden Ansprüche, wobei die in der zweiten Entspannungsstufe erhaltene Flüssigphase in den oberen Teil einer Kolonne geleitet wird und die in der zweiten Entspannungsstufe erhaltene Gasphase in den Sumpf der Kolonne geleitet wird, so dass Gasphase und Flüssigphase im Gegenstrom behandelt werden.

6. Verfahren nach Anspruch 5, wobei die am Kopf der Kolonne anfallende Gasphase durch Kondensation in eine Gasphase, welche im Wesentlichen unumgesetztes Synthesegas und unumgesetztes Olefin sowie das dem Olefin entsprechende Alkan enthält und in eine Flüssigphase, welche im Wesentlichen das Hydroformylierungsprodukt und geringe Mengen an nicht umgesetztem Olefin und gesättigten Kohlenwasserstoffen enthält, aufgetrennt wird.

7. Verfahren nach Anspruch 5 oder 6, wobei die am Sumpf der Kolonne anfallende Flüssigphase ganz oder teilweise in den Reaktor zurückgeführt wird.

8. Verfahren nach Anspruch 1, wobei die nach Kondensation erhaltene Gasphase ganz oder teilweise in den Reaktor zurückgeführt wird.

9. Verfahren nach einem der vorhergehenden Ansprüche, wobei die kontinuierliche Hydroformylierungsreaktion mit einem C₂-C₂₀-Olefin oder einem Gemisch davon durchgeführt wird.

10. Verfahren nach Anspruch 9, wobei als Olefin Propen eingesetzt wird.

## Claims

1. A process for working up a liquid output from a continuous hydroformylation, which comprises essentially aldehydes, high-boiling by-products, a homogeneously dissolved hydroformylation catalyst, unreacted olefins, low-boiling by-products and dissolved synthesis gas, wherein
a) the liquid hydroformylation output is depressurized in a first depressurization stage to a pressure which is from 2 to 20 bar below the reactor pressure and the pressure in the depressurization vessel is in the range from 2 to 40 bar, resulting in separation into a liquid phase and a gas phase, and the gas phase obtained in the first depressurization stage being recirculated to the reactor; and
b) the liquid phase obtained in the first depressurization stage is depressurized in a second depressurization stage to a pressure which is lower than the pressure of the first depressurization stage, resulting in separation into a liquid phase comprising essentially high-boiling by-products of the hydroformylation, the homogeneously dissolved hydroformylation catalyst and small amounts of hydroformylation product and unreacted olefin and a gas phase comprising essentially the major part of the hydroformylation product, unreacted olefin and low-boiling by-products.

2. The process according to claim 1, wherein the hydroformylation output is heated to a temperature which is from 5 to 50°C above the reaction temperature of the hydroformylation prior to the first depressurization stage.

3. The process according to claim 1 or 2, wherein the hydroformylation output is depressurized in the first depressurization stage to a pressure in the range from 3 to 40 bar.

4. The process according to any of the preceding claims, wherein the liquid phase obtained in the first depressurization stage is depressurized in the second depressurization stage to a pressure in the range from 0 to 10 bar.

5. The process according to any of the preceding claims, wherein the liquid phase obtained in the second depressurization stage is introduced into the upper part of a column and the gas phase obtained in the second depressurization stage is introduced at the bottom of the column so that gas phase and liquid phase are treated in countercurrent.

6. The process according to claim 5, wherein the gas phase obtained at the top of the column is separated by condensation into a gas phase which comprises essentially unreacted synthesis gas and unreacted olefin together with the alkane corresponding to the olefin and a liquid phase which comprises essentially the hydroformylation product and small amounts of unreacted olefin and saturated hydrocarbons.

7. The process according to claim 5 or 6, wherein the liquid phase obtained at the bottom of the column is recirculated wholly or partly to the reactor.

8. The process according to claim 1, wherein the gas phase obtained after condensation is recirculated wholly or partly to the reactor.

9. The process according to any of the preceding claims, wherein the continuous hydroformylation reaction is carried out using a C₂-C₂₀-olefin or a mixture thereof.

10. The process according to claim 9, wherein the olefin used is propene.

## Revendications

1. Procédé de traitement d'un extrait liquide d'une hydroformylation en continu, qui contient essentiellement des aldéhydes, des produits secondaires de point d'ébullition élevé, un catalyseur d'hydroformylation dissous de manière homogène, des oléfines n'ayant pas réagi, des produits secondaires de faible point d'ébullition et du gaz de synthèse dissous, dans lequel
a) l'extrait liquide de l'hydroformylation est détendu dans une première étape de détente, jusqu'à une pression qui se situe de 2 à 20 bar sous la pression de réaction et la pression dans le récipient de détente se situe dans une plage de 2 à 40 bar, une séparation en une phase liquide et une phase gazeuse se produisant, et la phase gazeuse obtenue dans la première étape de détente étant ramenée dans le réacteur, et
b) la phase liquide obtenue dans la première étape de détente est détendue dans une deuxième étape de détente, jusqu'à une pression qui est inférieure à la pression de la première étape de détente, une séparation se produisant en une phase liquide, qui contient essentiellement des produits secondaires de point d'ébullition élevé de l'hydroformylation, le catalyseur d'hydroformylation dissous de manière homogène et de faibles quantités de produit d'hydroformylation et de l'oléfine n'ayant pas réagi, et en une phase gazeuse, qui contient essentiellement la plus grande partie du produit d'hydroformylation, de l'oléfine n'ayant pas réagi et les produits secondaires de faible point d'ébullition.

2. Procédé selon la revendication 1, dans lequel l'extrait d'hydroformylation est chauffé avant la première étape de détente, jusqu'à une température qui se situe de 5 à 50°C au-dessus de la température de réaction de l'hydroformylation.

3. Procédé selon la revendication 1 ou 2, dans lequel, dans la première étape de détente, on détend jusqu'à une pression qui se situe dans l'intervalle allant de 3 à 40 bar.

4. Procédé selon l'une quelconque des revendications précédentes, dans lequel la phase liquide obtenue dans la première étape de détente est détendue dans la deuxième étape de détente, jusqu'à une pression située dans l'intervalle allant de 0 à 10 bar.

5. Procédé selon l'une quelconque des revendications précédentes, dans lequel la phase liquide obtenue dans la deuxième étape de détente est amenée dans la partie supérieure d'une colonne et la phase gazeuse obtenue dans la deuxième étape de détente est amenée au bas de la colonne, de sorte que la phase gazeuse et la phase liquide soient traitées à contre-courant.

6. Procédé selon la revendication 5, dans lequel la phase gazeuse obtenue en tête de colonne est séparée par condensation, en une phase gazeuse, qui contient essentiellement du gaz de synthèse n'ayant pas réagi et l'oléfine n'ayant pas réagi, ainsi que l'alcane correspondant à l'oléfine, et en une phase liquide, qui contient essentiellement le produit d'hydroformylation et de faibles quantités d'oléfine n'ayant pas réagi et d'hydrocarbures saturés.

7. Procédé selon la revendication 5 ou 6, dans lequel la phase liquide obtenue au bas de la colonne est partiellement ou complètement ramenée dans le réacteur.

8. Procédé selon la revendication 1, dans lequel la phase gazeuse obtenue après la condensation est partiellement ou complètement ramenée dans le réacteur.

9. Procédé selon l'une quelconque des revendications précédentes, dans lequel la réaction d'hydroformylation en continu est réalisée avec une oléfine en C₂-C₂₀ ou un mélange de celles-ci.

10. Procédé selon la revendication 9, dans lequel on met en oeuvre comme oléfine, le propène.
